# EUROPEAN PATENT SPECIFICATION

(11) **EP 1 871 338 B1**
(45) Date of publication and mention of the grant of the patent: **22.08.2012**
(21) Application number: 05750113.2
(22) Date of filing: 21.04.2005
(51) Int. Cl.: D06M 11/17, D06M 15/227, A61Q 15/00, A61K 8/26

(54) **METHOD AND COMPOSITION FOR OBTAINING ODOR-SUPPRESSING TEXTILE PRODUCTS AND TEXTILE PRODUCTS, NAMELY GARMENTS, THUS OBTAINED**
VERFAHREN UND ZUSAMMENSETZUNG ZUR GEWINNUNG EINES GERUCHSUNTERDRÜCKENDEN TEXTILPRODUKTS UND SO ERHALTENE TEXTILPRODUKTE, INSBESONDERE KLEIDUNGSSTÜCKE
PROCÉDÉ ET COMPOSITION POUR OBTENIR DES PRODUITS TEXTILES SUPPRIMANT LES ODEURS ET PRODUITS TEXTILES, À SAVOIR DES VÊTEMENTS, AINSI OBTENUS

(43) Date of publication of application: 02.01.2008
(73) Proprietor: Sicem Industriale S.p.A., 50019 Sesto Fiorentino (IT)
(72) Inventor: FRATINI, Marcello, I-58019 Monte Argentario (IT); SALINARO, Paolo, I-50019 Sesto Fiorentino (IT)
(74) Representative: Soldatini, Andrea
(86) International application number: PCT/IT2005/000237
(87) International publication number: WO 2006/111991

(56) References cited:
- EP-A- 0 006 739
- WO-A-02/49591
- GB-A- 1 385 051
- US-A- 5 103 500
- US-A1- 2003 135 932
- US-B1- 6 592 858
- PATENT ABSTRACTS OF JAPAN vol. 2003, no. 04, 2 April 2003 (2003-04-02) & JP 2002 370911 A (DAIKYO KAGAKU KK), 24 December 2002 (2002-12-24)

## Description

### Field of the invention

The present invention relates to a method for obtaining garments, and more generally textile products, capable of suppressing the formation of the bad smell caused by sweating. It therefore extends also to textile products obtained with the method.

### Description of the prior art

It is well known that human body can emanate bad smell due to sweating. This phenomenon is even more perceptible when, especially with hot weather, the conditions are favorable for the birth and the development of bacteria that decompose the body secretions, transform them in bad-smelling substances. In fact, it is common experience that the sweat is practically odor-free as soon as it is produced by the body, and that the longer the body remains in contact with the sweat and with the garments impregnated with it, the more unpleasant is the odor developed.

For suppressing the development of the bacteria that cause the bad odor, the use of aluminium salts, namely aluminium chloride, as a deodorizing agent. Various ways of applying said deodorizing agent directly to a fabric have also been proposed, in order to obtain a garment that is intrinsically provided with odor-suppressing properties (see for instance the published PCT international patent application n. WO02/49591, or JP2002370911).

However, these proposals have not resulted satisfactory, basically due to two kinds of problems: the impregnation of the textile fiber with the deodorizing agent changes the physical characteristics of the fiber itself, with consequent worsening of its quality; the association of the deodorizing agent to the textile fiber is not sufficiently stable, thereby the odor-suppressing properties of the garment are lost after only a few washings.

### Summary of the invention

The applicant has now identified a fully effective solution of the problems outlined above thanks to a method capable of binding an aluminium chloride based deodorizing agent to a textile fiber, without in any way impairing the quality of the latter and above all ensuring that the association between the agent and the fiber resists to quite a lot of washings, and thus that a garment can be obtained that maintains its odor-suppressing properties for a long time.

The essential features of the method according to the invention are defined in the first of the appended claims. The dependent claims specify advantageous-embodiments of the method.

### Description of preferred embodiments

The characteristics and advantages of the method and composition for obtaining odor-suppressing textile products and textile products, namely garments, thus obtained according to the present invention will be brought out more clearly by the following description of its embodiments, which is given purely by way of example and is not to be taken as limitative in any way.

According to the invention, a deodorizing composition is prepared, to be used for treating the textile product destined to be worn on a user's body, both a finished product (garment) and in nature of a raw or semifinished material (fabric, yarn etc.).

A simple example of a deodorizing composition for applying the deodorizing active principle (agent) to garments made of cotton consists of an aqueous bath prepared as follows:
- 4% in volume of a 20% aqueous solution of aluminium chloride;
- 5 ÷ 20 g/l of a polymeric binder, such as acrylic resin;
- 10 ÷ 20 g/l of a cationic surfactant, e.g. a common quaternary-ammonium-based fixative for dyes;
- 2 ÷ 5 g/l of a softener, e.g. a perfumed Henkel^{®} softener.

The garments to be treated are immersed for a few minutes (no more than 20 minutes) in the bath prepared in this manner, which is brought to a temperature of about 90°C. When a fibrous material containing wool is involved, the polymeric binder will preferably be a silicon based resin, and the temperature of the bath around 50°C.

The treatment is completed with a phase of rinsing and drying with hot air at a temperature suitable for the polymerization of the resin (normally variable between 70° and 180°). The treatment time and temperature may however be varied according to the particular machine employed. The indications provided above are considered to be optimal in the case of treatment with, for example, a centrifugal washing machine.

In a different reduction to practice of the method according to the invention, the active principle can be applied prior to the actual manufacturing of the garments, i.e. to the fabrics from which these garments are to be made. This can be done, for example, in a continuous process by carrying out the impregnation in a foulard machine with a bath containing 5 ÷ 10 g/l of the active principle in emulsified form with a non-ionic surfactant, 3 ÷ 5 g/l of emulsified acrylic resin, in a pH made slightly acid by means of acetic acid (pH=5) in case of wool-based fabrics, or in a neutral pH in case of fabrics with a cellulose base. After wringing the fabric, it is dried in a "Rameuse" machine at the temperature which is necessary to assure the polymerization of the acrylic resin.

In the compositions suggested above the polymeric binder obviously has the fundamental function of fixing the active principle to the textile fiber, holding it and thus making the association resistant to subsequent washings of the textile product. The aluminium chloride is released very slowly, thus assuring its deodorizing action in the course of time. Neither the silicon resin nor the acrylic resin causes any appreciable alteration of the softness characteristics of the fiber and they are therefore particularly suitable for this purpose. Other types of resins may however be used - e. g. butadiene, polyurethane, polyamide or acrylonitrile based resins - even combined in appropriate proportions.

In case of materials with a woolen base it is preferable to use resins capable of being polymerized at low temperatures, like those with radical-type polymerization mechanisms. In any case, the softener may serve to attenuate a possible stiffening effect deriving from the presence of the resin.

Application to textile fibers, for example and typically in accordance with the modalities described above, represents a particularly advantageous reduction to practice of the invention, because it assures an optimal effectiveness of the deodorizing action without in any way altering either the appearance or the original softness of the supporting materials, which will also remain wholly free of smell.

The combination of the aluminium chloride with the polymeric binder results in a stable adhesion to the textile support, ensuring that the deodorizing agent is released persistently in the course of time, and this even after a number of washings. Garments treated in the manner just described, when worn, suppress the development of bad odors from the body, even after an intense and prolonged sweating. Experimental tests carried out on people particularly prone to this kind of problem, who for this purpose were made to wear garments treated according to the invention, demonstrated the complete disappearance of the bad smell. Moreover, repeated washings of the garments did not bring out any perceptible decay of the deodorizing properties. No allergic manifestation of any kind to the detriment of the wearers were revealed by any of the tests. It should also be noted that the deodorizing principle in question, apart from being inert, does not interact with the sebaceous secretion and is therefore absolutely tolerable from a hygienic and sanitary point of view.

Application to textile fibers, for example and typically in accordance with the modalities described above, assures an optimal effectiveness of the deodorizing action without in any way altering either the appearance or the original softness of the supporting materials, which will also remain wholly free of any particular smell. However, this application can be carried out with various modalities, especially in accordance with the variations of the material for which it is intended and therefore also of the machines that are employed. For example, the latter could include the so-called "Dutch machines", where the movement of the bath is more gentle and thus avoids the physical alteration of materials made of wool fibers.

Variants and/or modifications can be brought to the method and composition for obtaining odor-suppressing textile products and textile products, namely garments, thus obtained without thereby departing from the scope of the invention itself as defined in the appended claims.

## Claims

1. A method for obtaining odor-suppressing textile products intended to be worn on the human body making use of an active deodorizing principle consisting of aluminium chloride or, in any case, a substance capable of liberating aluminium chloride, said products being partially or completely treated with a composition comprising, besides said active principle, a polymeric binder comprising at least one resin chosen from the following group: acrylic-based resin, silicone-based resin, butadiene-based resin, polyurethane-based resin, polyamide-based resin and acrylonitrile-based resin; wherein said active principle and said resin are distributed in an aqueous bath in which said products are immersed, whereby said active principle is stably fixed to said products and released gradually in the course of time, **characterized in that** said aqueous bath comprises 4% in volume of a 20% aqueous solution of said active principle, said polymeric binder having a concentration comprised between 5 and 20 g/l, said aqueous bath comprising also a cationic surfactant and a softener having a concentration of, respectively, between 10 and 20 g/l and between 2 and 5 g/l.

2. The method according to claim 1, wherein said aqueous bath is brought to a temperature of at least 40°C.

3. A composition for partially or completely treating textile products intended to be worn on the human body making use of an active deodorizing principle consisting of aluminium chloride or, in any case, a substance capable of liberating aluminium chloride, the composition comprising, in addition to said active principle, a polymeric binder comprising at least one resin chosen from the following group: acrylic-based resin, silicone-based resin, butadiene-based resin, polyurethane-based resin, polyamide-based resin and acrylonitrile-based resin; wherein said active principle and said resin are distributed in an aqueous bath in which said products are to be immersed, whereby said active principle is stably fixed to said products and released gradually in the course of time, the composition being **characterized in that** it comprises 4% in volume of a 20% aqueous solution of said active principle, said polymeric binder having a concentration comprised between 5 and 20 g/l, said aqueous bath comprising also a cationic surfactant and a softener having a concentration of, respectively, between 10 and 20 g/l and between 2 and 5 g/l.

4. Textile products partially or totally impregnated or treated with a composition according to claim 3.

## Patentansprüche

1. Verfahren zum Erhalten von Geruch unterdrückenden textilen Produkten, die vorgesehen sind, um auf dem menschlichen Körper getragen zu werden, unter Verwendung eines desodorierenden Wirkstoffes, bestehend aus Aluminiumchlorid oder in jedem Fall einer Substanz, die zur Freisetzung von Aluminiumchlorid geeignet ist, wobei die Produkte teilweise oder vollständig mit einer Zusammensetzung behandelt werden, die neben dem Wirkstoff ein polymeres Bindemittel enthält, das wenigstens ein Harz enthält, das aus der folgenden Gruppe ausgewählt ist: Acryl-basiertes Harz, Silikon-basiertes Harz, Butadien-basiertes Harz, Polyurethanbasiertes Harz, Polyamid-basiertes Harz und Acrylnitrilbasiertes Harz; wobei der Wirkstoff und das Harz in einem wässrigen Bad verteilt sind, in das die Produkte getaucht werden, wodurch der Wirkstoff stabil an den Produkten fixiert und graduell im Laufe der Zeit freigegeben wird, **dadurch gekennzeichnet, dass** das wässrige Bad 4 Volumen-% einer 20 prozentigen wässrigen Lösung des Wirkstoffes enthält, wobei das polymere Bindemittel eine Konzentration hat, die zwischen 5 und 20 g/l liegt, wobei das wässrige Bad auch ein kationisches Tensid und einen Weichmacher enthält, die eine Konzentration von entsprechend zwischen 10 und 20 g/l bzw. zwischen 2 und 5 g/l haben.

2. Verfahren nach Anspruch 1, wobei das wässrige Bad auf eine Temperatur von mindestens 40 °C gebracht wird.

3. Zusammensetzung zur teilweisen oder vollständigen Behandlung von textilen Produkten, die vorgesehen sind, um auf dem menschlichen Körper getragen zu werden, unter Verwendung eines desodorierenden Wirkstoffes, bestehend aus Aluminiumchlorid oder in jedem Fall einer Substanz, die zur Freisetzung von Aluminiumchlorid geeignet ist, wobei die Zusammensetzung neben dem Wirkstoff ein polymeres Bindemittel enthält, das wenigstens ein Harz enthält, das aus der folgenden Gruppe ausgewählt ist: Acryl-basiertes Harz, Silikon-basiertes Harz, Butadien-basiertes Harz, Polyurethan-basiertes Harz, Polyamid-basiertes Harz und Acrylnitril-basiertes Harz; wobei der Wirkstoff und das Harz in einem wässrigen Bad verteilt sind, in das die Produkte einzutauchen sind, wodurch der Wirkstoff stabil an den Produkten fixiert und graduell im Laufe der Zeit freigegeben wird, **dadurch gekennzeichnet, dass** sie 4 Volumen-% einer 20 prozentigen wässrigen Lösung des Wirkstoffes enthält, wobei das polymere Bindemittel eine Konzentration hat, die zwischen 5 und 20 g/l liegt, wobei das wässrige Bad auch ein kationisches Tensid und einen Weichmacher enthält, die eine Konzentration von entsprechend zwischen 10 und 20 g/l bzw. zwischen 2 und 5 g/l haben.

4. Textile Produkte, die teilweise oder vollständig imprägniert oder behandelt sind mit einer Zusammensetzung nach Anspruch 3.

## Revendications

1. Procédé pour obtenir des produits textiles supprimant les odeurs qui sont destinés à être portés sur un corps humain, utilisant un principe actif désodorisant consistant en du chlorure d'aluminium ou, en tous cas, en une substance apte à libérer du chlorure d'aluminium, lesdits produits étant partiellement ou complètement traités avec une composition comprenant, en plus du dit principe actif, un liant polymère comprenant au moins une résine choisie dans le groupe suivant : résine à base d'acrylique, résine à base de silicone, résine à base de butadiène, résine à base de polyuréthane, résine à base de polyamide et résine à base d'acrylonitrile ; où ledit principe actif et ladite résine sont distribués dans un bain aqueux dans lequel lesdits produits sont immergés, par lequel ledit principe actif est fixé de façon stable sur lesdits produits et libéré de façon graduelle au cours du temps, **caractérisé en ce que** ledit bain aqueux comporte 4% en volume d'une solution aqueuse à 20% du dit principe actif, ledit liant polymère ayant une concentration comprise entre 5 et 20 g/l, ledit bain aqueux comprenant également un surfactant cationique et un adoucissant ayant une concentration respectivement entre 10 et 20 g/l et entre 2 et 5 g/l.

2. Le procédé selon la revendication 1, où ledit le bain aqueux est porté à une température d'au moins 40°C.

3. Une composition pour traiter partiellement ou complètement des produits textiles destinés à être portés sur le corps humain utilisant un principe actif désodorisant consistant en du chlorure d'aluminium ou, en tous cas, en une substance apte à libérer du chlorure d'aluminium, la composition comportant, en plus du dit principe actif, un liant polymère comprenant au moins une résine choisie dans le groupe suivant : résine à base d'acrylique, résine à base de silicone, résine à base de butadiène, résine à base de polyuréthane, résine à base de polyamide et résine à base d'acrylonitrile ; où ledit principe actif et ladite résine sont distribués dans un bain aqueux dans lequel lesdits produits doivent être immergés, par lequel ledit principe actif est fixé de façon stable sur lesdits produits et libéré de façon graduelle au cours du temps, la composition étant **caractérisée en ce qu'**elle comporte 4% en volume d'une solution aqueuse à 20% du dit principe actif, ledit liant polymère ayant une concentration comprise entre 5 et 20 g/l, ledit bain aqueux comportant également un surfactant cationique et un adoucissant ayant une concentration respectivement entre 10 et 20 g/l et entre 2 et 5 g/l.

4. Produits textiles partiellement ou totalement imprégnés ou traités avec une composition selon la revendication 3.
